# EUROPEAN PATENT APPLICATION

(11) **EP 4 653 521 A1**
(43) Date of publication of application: **26.11.2025**
(21) Application number: 25176982.4
(22) Date of filing: 16.05.2025
(51) Int. Cl.: C12M 1/00, C12M 3/00, A61M 1/14, B01L 3/00

(54) **PANEL CIRCUIT AND CELL PROCESSING DEVICE**

(30) Priority: 22.05.2024 JP 2024083608
(71) Applicant: FUJIFILM Corporation, Tokyo 106-8620 (JP)
(72) Inventor: KAMEI, Shota, Ashigarakami-gun, Kanagawa, 258-8577 (JP); OBA, Takahiro, Ashigarakami-gun, Kanagawa, 258-8577 (JP)
(74) Representative: Hoffmann Eitle

(57) **Abstract**

Provided are a panel circuit and a cell processing device capable of easily and accurately attaching a member including a flexible tube.

A panel circuit includes a resin panel having a first groove portion on a first surface, and a flexible tube that is fitted into the first groove portion to be guided by the first groove portion. a portion of the second surface of the resin panel opposite to the first surface, on which the first groove portion is formed, protrudes from the other portion of the second surface, and the most part of the tube is fitted into the first groove portion.

## Description

### BACKGROUND OF THE INVENTION

### 1. Field of the Invention

The disclosed technology relates to a panel circuit and a cell processing device.

### 2. Description of the Related Art

The following technology is known as a technology related to positioning of a tube through which a liquid flows. For example, JP2018-143365A describes a panel circuit including a flexible tube that constitutes at least a part of a liquid circuit of a body fluid processing device and a panel that holds a part of the tube.

### SUMMARY OF THE INVENTION

In order to increase the production quantity of the regenerative medicine product, the automation of a part or all of the manufacturing process is being promoted. For example, in an automated cell processing device that is used for manufacturing regenerative medicine products, cell processing members, such as a filter, a filtration device, a centrifuge tube, and a bag, are connected to each other via a flexible tube (hereinafter, referred to as a tube assembly) and the connected members are attached to a housing. In regenerative medicine using autologous cells, since the tube assembly is operated in a single-use manner, the tube assembly is frequently attached and detached.

In the tube assembly, tubes or tubes and other members are likely to be entangled with each other, and handling of the tube assembly is complicated. In addition, in a case where the tube assembly is attached to the cell processing device, it is difficult to visualize the attachment state (the arrangement of the respective members) of the tube assembly, and there is a concern that it takes a long time to perform the attachment work or work mistakes occurs. Therefore, as in the technology described in JP2018-143365A, the mountability of the device is improved by holding a part of the tube on the panel. However, in this case, a portion of the tube that is not fixed to the panel protrudes out of the panel surface, which causes the tube to be entangled or separated due to vibration in a case of transportation, tension in a case of being mounted on the device, or the like.

The disclosed technology has been made in view of the above-mentioned points, and an object of the disclosed technology is to suppress a risk that a flexible tube attached to a panel is separated from the panel due to an external force and to easily and accurately attach a member including the tube.

The panel circuit includes a resin panel having a first groove portion on a first surface, and a flexible tube fitted into the first groove portion to be guided by the first groove portion. a portion of the second surface of the resin panel opposite to the first surface, on which the first groove portion is formed, protrudes from the other portion of the second surface, and the most part of the tube is fitted into the first groove portion.

The resin panel may further have a second groove portion into which the tube is not fitted. The first groove portion and the second groove portion may have a bent portion or a curved portion. The first groove portion may have a portion extending in a first direction and a portion extending in a second direction intersecting the first direction. The second groove portion may have a portion extending in a first direction and a portion extending in a second direction intersecting the first direction.

The second groove portion may have a larger width than the first groove portion. The second groove portion may have a greater depth than the first groove portion. A thickness of the resin panel may be 10 mm or less. The resin panel may have light transmittance.

The panel circuit may further include at least one cell processing member connected to the tube. The cell processing member may include any one or more of a filter, a centrifuge tube, a bag, or a bottle.

The cell processing device according to an embodiment of the disclosed technology includes the above-described panel circuit, at least one pump that performs feeding of a liquid flowing inside the tube, at least one pinch valve that switches between closing and opening of the tube, and a liquid sensor that senses the liquid flowing inside the tube.

The pump, the pinch valve, and the liquid sensor may be provided on the same surface of a housing surface, the resin panel may have a plurality of hole portions and may be attached to the housing surface in a state in which any one of the pump, the pinch valve, or the liquid sensor is inserted into any one of the plurality of hole portions.

According to the disclosed technology, it is possible to easily and accurately attach a member including a flexible tube.

### BRIEF DESCRIPTION OF THE DRAWINGS

Fig. 1 is a view showing an example of a configuration of a tube assembly according to an embodiment of the disclosed technology.
Fig. 2 is a plan view showing an example of a configuration of a resin panel according to the embodiment of the disclosed technology.
Fig. 3 is a cross-sectional view taken along line 3-3 in Fig. 2.
Fig. 4 is a plan view showing an example of a configuration of a panel circuit according to the embodiment of the disclosed technology.
Fig. 5 is a cross-sectional view of a resin panel according to a comparative example.
Fig. 6 is a cross-sectional view showing an example of a configuration of a resin panel according to another embodiment of the disclosed technology.
Fig. 7 is a view showing an example of a configuration of a cell processing device according to the embodiment of the disclosed technology.
Fig. 8 is a view showing an example of a configuration of a cell processing device in a state where a panel circuit according to the embodiment of the disclosed technology is attached.
Fig. 9 is a flowchart showing an example of a life cycle of a panel circuit according to the embodiment of the disclosed technology.

### DESCRIPTION OF THE PREFERRED EMBODIMENTS

Hereinafter, an example of an embodiment of the disclosed technology will be described with reference to the drawings. It should be noted that, the same or equivalent components and portions in each of the drawings are assigned by the same reference numerals, and the overlapping description will be omitted.

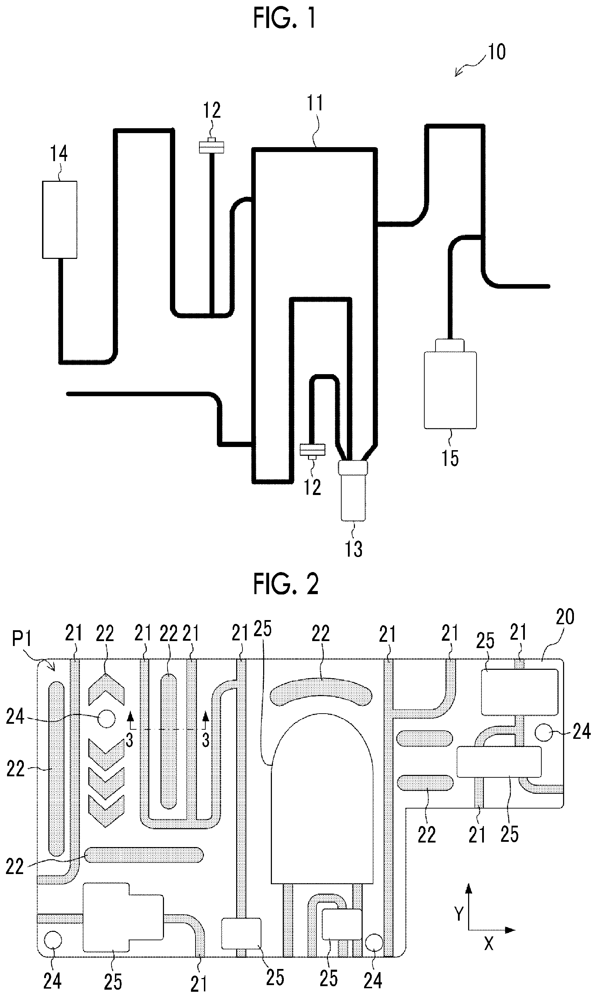

Fig. 1 is a view showing an example of a configuration of a tube assembly 10 according to an embodiment of the disclosed technology. The tube assembly 10 is a component of a cell processing device described later, and has a flexible tube 11 and a plurality of cell processing members connected to the tube 11. Fig. 1 shows a filter 12, a centrifuge tube 13, a bag 14, and a bottle 15 as examples of the cell processing members. The tube 11 may be made of, for example, a synthetic resin such as a fluororesin, polyethylene, or polystyrene. The tube 11 has a plurality of branch points, and the cell processing member, such as a filter 12, a centrifuge tube 13, a bag 14, and a bottle 15, is connected to the distal end of a portion extending from each branch point of the tube 11. The filter 12 is provided, for example, at an end portion that serves as an air vent of the tube 11, and prevents the infiltration of contaminants into the tube 11. The centrifuge tube 13, the bag 14, and the bottle contain a liquid such as a cell suspension and a culture medium. The tube assembly 10 is attached to a cell processing device described later.

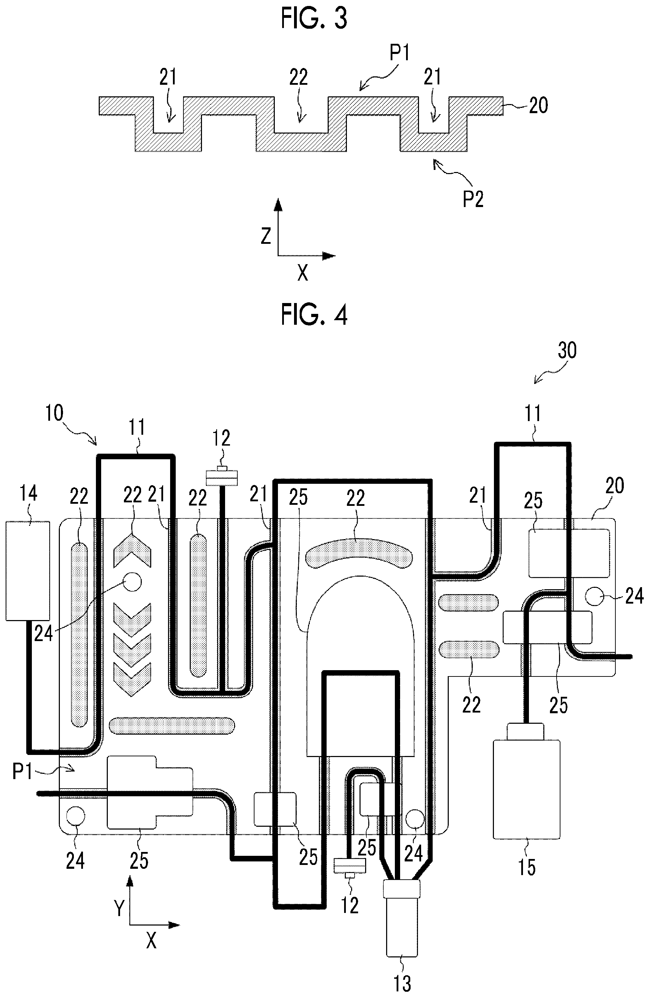

Fig. 2 is a plan view showing an example of a configuration of a resin panel 20 according to the embodiment of the disclosed technology, and shows a first surface P1 of the resin panel 20. Fig. 3 is a cross-sectional view taken along line 3-3 in Fig. 2. The resin panel 20 is a plate-like member that assists in attachment of the tube assembly 10 to the cell processing device. The resin panel 20 is made of a synthetic resin such as polyethylene terephthalate (PET). The planar size of the resin panel 20 is not particularly limited, but a relatively large size corresponding to the scale of the tube assembly 10 is assumed. The dimension of the resin panel 20 in the X direction in the drawing may be, for example, about 500 mm, and the dimension in the Y direction in the drawing may be, for example, about 300 mm. The resin panel 20 is made thin in order to reduce the weight. The thickness of the resin panel 20 is preferably 10 mm or less and more preferably 5 mm or less.

The resin panel 20 has a first groove portion 21 and a second groove portion 22 on the first surface P1. The first groove portion 21 is a linear recess portion that guides the path of the tube 11. Each portion of the tube 11 is fitted into the first groove portion 21 to be guided by the first groove portion 21.

Fig. 4 is a plan view showing an example of a configuration of a panel circuit 30 configured by fitting the tube 11 into the first groove portion 21. The tube 11 fitted into the first groove portion 21 is held in the resin panel 20 by being interposed between the side walls of the first groove portion 21. The first groove portion 21 is formed with a width and a depth such that the fitted tube 11 does not easily fall off. The tube 11 is guided by the first groove portion 21 to position the path of the tube 11, and accordingly, the filter 12, the centrifuge tube 13, the bag 14, and the bottle 15 are positioned. The most part of the tube 11 (80% or more of the tube length extending on the main surface of the resin panel 20) is fitted into the first groove portion 21.

The tube 11 is not fitted into the second groove portion 22. The second groove portion 22 is a structure for increasing the stiffness of the resin panel 20. By forming the second groove portion 22 in the region of the resin panel 20 where the first groove portion 21 is not formed, it is possible to increase the stiffness of the resin panel 20 without bias. The first groove portion 21 also contributes to the improvement of the stiffness of the resin panel 20. That is, the first groove portion 21 has both a function of guiding the tube 11 and a function of increasing the stiffness of the resin panel 20. Meanwhile, the second groove portion 22 has a structure for exclusively increasing the stiffness of the resin panel 20. Since the resin panel 20 has not only the first groove portion 21 but also the second groove portion 22, it is possible to secure sufficient stiffness even though the resin panel 20 is thin.

The first groove portion 21 and the second groove portion 22 each have a portion extending in the X direction in the drawing and a portion extending in the Y direction in the drawing. In addition, each of the first groove portion 21 and the second groove portion 22 has a bent portion or a curved portion. As a result, the stiffness of the resin panel 20 can be further increased, and the thinning and weight reduction of the resin panel 20 can be achieved.

The first groove portion 21 and the second groove portion 22 are formed by a vacuum forming method. The vacuum forming method is a method of forming a resin sheet by placing a heated and plasticized resin sheet on a mold and sucking the resin sheet to the mold by bringing a space between the resin sheet and the mold into a vacuum state. By forming the first groove portion 21 and the second groove portion 22 by a vacuum forming method, as shown in Fig. 3, a portion of the second surface P2 of the resin panel 20, in which the first groove portion 21 and the second groove portion 22 are formed, has a structure protruding from the other portion of the second surface P2. In the resin panel 20, the thickness of a portion in which the first groove portion 21 and the second groove portion 22 are formed and the thickness of the other portion are substantially the same.

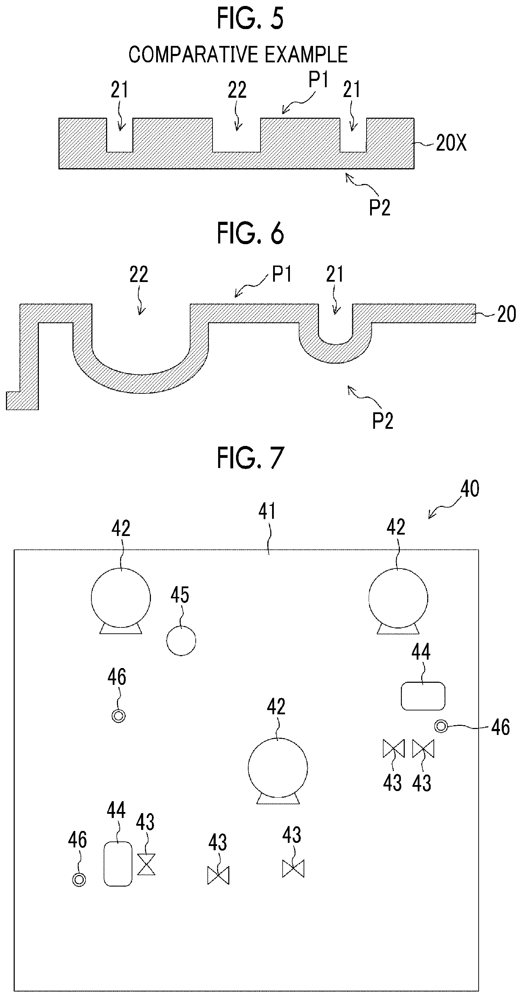

Here, Fig. 5 is a cross-sectional view of a resin panel 20X according to a comparative example in which the first groove portion 21 and the second groove portion 22 are formed by cutting, injection molding, or the like. In the resin panel 20X, the second surface P2 is flat. According to the resin panel 20X, since it is necessary to have a thickness having a larger dimension than the depth dimensions of the first groove portion 21 and the second groove portion 22, the weight is increased and the cost is high. On the other hand, in the resin panel 20 (see Fig. 3) according to the embodiment of the disclosed technology, since the first groove portion 21 and the second groove portion 22 are formed by a vacuum forming method, the thickness of the resin panel 20 can be reduced, and it is possible to achieve weight reduction and cost reduction.

As shown in Fig. 3, the cross-sectional shapes of the first groove portion 21 and the second groove portion 22 may be rectangular. The second groove portion 22 may have a larger width than the first groove portion 21. In addition, as shown in Fig. 6, the bottom surface of each of the first groove portion 21 and the second groove portion 22 may be a curved surface. The second groove portion 22 may have a greater depth than the first groove portion 21.

The resin panel 20 has a hole portion 24 into which a positioning shaft is inserted in a case where the resin panel 20 is attached to the cell processing device. In addition, the resin panel 20 has a plurality of hole portions 25 for disposing various devices such as a pump provided in the cell processing device in the panel surface.

Fig. 7 is a diagram showing an example of a configuration of the cell processing device 40. The cell processing device 40 has a function of automatically performing predetermined processing on cells, and is used, for example, for manufacturing a regenerative medicine product. The regenerative medicine product is a product created by processing such as culturing living cells or tissues of a person or an animal, and is used for reconstructing, repairing, or forming a structure or function of a body, for treating or preventing a disease, or for being introduced into a human cell for gene therapy. The regenerative medicine products include, for example, cultured cells, cultured skin, and cultured cartilage.

The cell processing device 40 has a pump 42, a pinch valve 43, a liquid sensor 44, a pressure gauge 45, and a positioning shaft 46, which are provided on a front surface of a housing 41 thereof. That is, the pump 42, the pinch valve 43, the liquid sensor 44, the pressure gauge 45, and the positioning shaft 46 are provided on the same surface of the housing surface. The pump 42 feeds a liquid (for example, a cell suspension) flowing inside the tube 11. The pinch valve 43 switches between the closing and the opening of the tube 11. The liquid sensor 44 senses a liquid flowing inside the tube 11. The pressure gauge 45 monitors the pressure inside the tube 11. The positioning shaft 46 extends in a vertical direction from the front surface of the housing 41.

The cell processing device 40 is used in a state in which the respective constituent devices are connected to the tube assembly 10. The attachment of the tube assembly 10 to the cell processing device 40 is assisted by the resin panel 20. That is, the panel circuit 30 configured by fitting the tube 11 into the first groove portion 21 of the resin panel 20 is attached to the front surface of the housing 41 of the cell processing device 40.

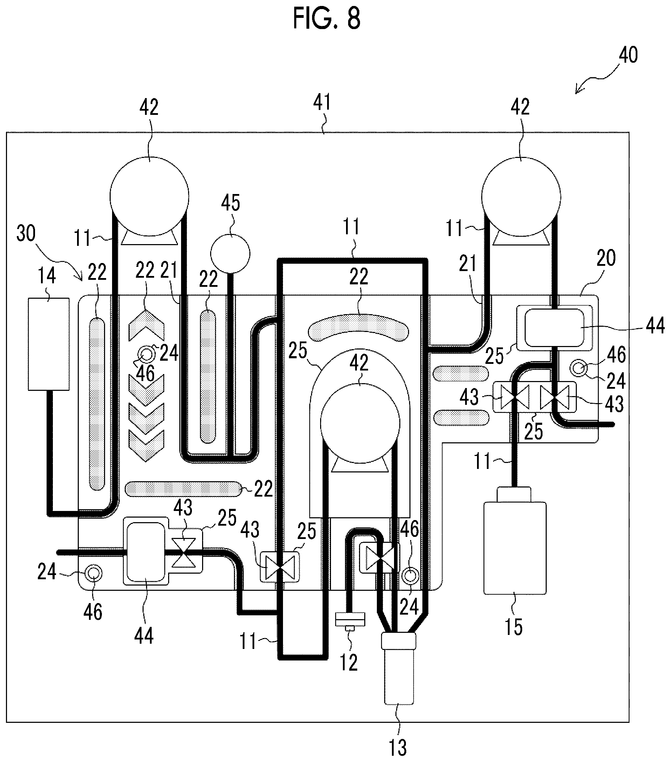

Fig. 8 is a diagram showing an example of a configuration of the cell processing device 40 in a state where the panel circuit 30 is attached. The positioning shaft 46 is inserted into the hole portion 24 of the resin panel 20, whereby the panel circuit 30 is aligned with the cell processing device 40. In a state where the panel circuit 30 is aligned, the pump 42, the pinch valve 43, and the liquid sensor 44 are inserted into the hole portion 25 of the resin panel 20, and these constituent devices are disposed in the panel surface. In a state where the panel circuit 30 is attached to the cell processing device 40, the constituent members (the filter 12, the centrifuge tube 13, the bag 14, and the bottle 15) of the tube assembly 10 are disposed at positions that match the constituent devices (the pump 42, the pinch valve 43, and the liquid sensor 44) of the cell processing device 40.

In order to improve workability in a case where the panel circuit 30 is attached to the cell processing device 40, it is preferable that the respective constituent devices provided on the front surface of the housing 41 through the resin panel 20 can be visually recognized. Therefore, the resin panel 20 is preferably transparent. By using, for example, PET as the material of the resin panel 20, it is possible to form the resin panel 20 that can be formed by a vacuum forming method and has light transmittance.

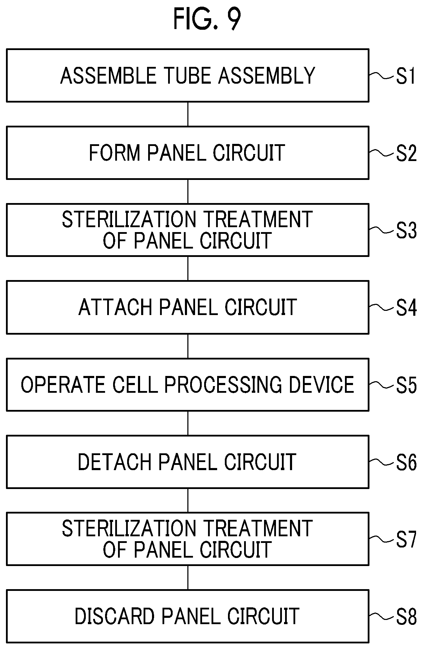

Fig. 9 is a diagram showing an example of a life cycle of the panel circuit 30. In step S1, the tube assembly 10 is assembled. That is, the cell processing member including the filter 12, the centrifuge tube 13, the bag 14, and the bottle 15 is connected to the tube 11.

In step S2, the panel circuit 30 is formed. The panel circuit 30 is formed by fitting the tube 11 into the first groove portion 21 of the resin panel 20. The tube 11 is guided by the first groove portion 21 to position the tube 11, and accordingly, the filter 12, the centrifuge tube 13, the bag 14, and the bottle 15 are positioned. Since the tube 11 is held by the linear first groove portion 21 that guides the path of the tube 11, the risk of the tube 11 being separated from the resin panel 20 is suppressed. In addition, the risk of the tube 11 being incompletely fitted into the first groove portion 21 is suppressed. That is, in a case where a portion, in which the tube 11 is not appropriately fitted into the first groove portion 21, is generated, the operator can easily notice this.

In step S3, the panel circuit 30 is subjected to a sterilization treatment. After the sterilization treatment, the inside of the tube assembly 10 is blocked from the outside, so that the inside of the tube assembly 10 is kept in a sterile state.

In Step S4, the panel circuit 30 is attached to the cell processing device 40. The panel circuit 30 is attached to the front surface of the housing 41 in which the constituent devices of the cell processing device 40 including the pump 42, the pinch valve 43, and the liquid sensor 44 are collectively provided. The positioning shaft 46 protruding from the front surface of the housing 41 is inserted into the hole portion 24 of the resin panel 20, thereby aligning the panel circuit 30 with respect to the cell processing device 40. Since the resin panel 20 has stiffness, the resin panel 20 is not warped or bent in the attachment of the panel circuit 30. As a result, the relative positional relationship between the respective constituent members of the tube assembly 10 is maintained in a fixed state. In a state where the panel circuit 30 is attached to the cell processing device 40, the constituent members (the filter 12, the centrifuge tube 13, the bag 14, and the bottle 15) of the tube assembly 10 are disposed at positions that match the constituent devices (the pump 42, the pinch valve 43, and the liquid sensor 44) of the cell processing device 40. Since the panel circuit 30 is thin and light, it is possible to reduce the burden of the attachment work of the panel circuit 30.

In step S5, the cell processing device 40 is operated. A liquid such as a cell suspension transferred by the pump 42 flows inside the tube assembly 10, and thus the cells are subjected to predetermined processing.

In Step S6, the panel circuit 30 is detached from the cell processing device 40.

In step S7, the panel circuit 30 is subjected to a sterilization treatment. In a case where the resin panel 20 is large and it is difficult to accommodate the resin panel 20 in a sterilizer such as an autoclave, in a state of the tube assembly 10 being attached to the resin panel 20, the tube assembly 10 may be detached from the resin panel 20 and sterilized in a state where the tube assembly 10 is alone. In step S8, the panel circuit 30 is discarded.

As described above, the tube 11 is fitted into the first groove portion 21 to be guided by the first groove portion 21. As a result, the tube 11 is positioned, and accordingly, the filter 12, the centrifuge tube 13, the bag 14, and the bottle 15 are positioned. That is, by disposing the tube 11 on the resin panel 20 according to the guide of the first groove portion 21, it is possible to set a relative positional relationship between the respective constituent members of the tube assembly 10 to an appropriate state. In addition, the tube assembly 10 can be attached to the cell processing device 40 while maintaining an appropriate relative positional relationship between the respective constituent members of the tube assembly 10.

Therefore, with the panel circuit 30 according to the embodiment of the disclosed technology, in a case where the tube assembly 10 is attached to the cell processing device 40, the tubes or the tubes and other members do not become entangled with each other, and it is possible to eliminate the complication of handling of the tube assembly 10. In addition, it is possible to easily realize a state in which the relative positional relationship between the respective constituent members of the tube assembly 10 is in an appropriate state, it is possible to shorten the work time required for the attachment of the tube assembly 10 to the cell processing device 40, and it is possible to suppress the occurrence of work mistakes. That is, according to the panel circuit 30, it is possible to easily and accurately attach the member including the flexible tube. Further, according to the panel circuit 30, the most part of the tube 11 (80% or more of the tube length extending on the main surface of the resin panel 20) is fitted into the first groove portion 21. By minimizing the portion of the tube 11 that protrudes out of the surface of the resin panel 20, it is possible to suppress the risk that the tube 11 is separated from the resin panel 20 due to an external force.

In addition, in the resin panel 20, since the first groove portion 21 and the second groove portion 22 are formed by a vacuum forming method, the thickness of the resin panel 20 can be reduced, and it is possible to reduce the weight and the cost.

In addition, since the resin panel 20 has not only the first groove portion 21 into which the tube 11 is fitted but also the second groove portion 22 into which the tube 11 is not fitted, it is possible to secure sufficient stiffness even though the resin panel 20 is thin.

The first groove portion 21 and the second groove portion 22 each have a portion extending in the X direction in the drawing and a portion extending in the Y direction in the drawing. In addition, each of the first groove portion 21 and the second groove portion 22 has a bent portion or a curved portion. As a result, the stiffness of the resin panel 20 can be further increased, and the thinning and weight reduction of the resin panel 20 can be achieved.

In addition, according to the cell processing device 40, since the constituent devices of the cell processing device 40 including the pump 42, the pinch valve 43, and the liquid sensor 44 are collectively provided on the entire surface of the housing 41, workability in a case where the tube assembly 10 is attached to the cell processing device 40 can be improved. Meanwhile, in this case, it is assumed that the scale of the panel circuit 30 is large, but the weight of the resin panel 20 is reduced while maintaining the stiffness, so that it is possible to avoid the deterioration of workability.

Regarding the above embodiments, the following supplementary notes are further disclosed.

### Supplementary Note 1

A panel circuit comprising:
a resin panel having a first groove portion on a first surface;
a flexible tube that is fitted into the first groove portion to be guided by the first groove portion,
wherein a portion of a second surface of the resin panel opposite to the first surface, which corresponds to the first groove portion, protrudes from the other portion of the second surface, and
a most part of the tube is fitted into the first groove portion.

### Supplementary Note 2

The panel circuit according to Supplementary Note 1,
wherein the resin panel further has a second groove portion into which the tube is not fitted.

### Supplementary Note 3

The panel circuit according to Supplementary Note 2,
wherein the first groove portion and the second groove portion have a bent portion or a curved portion.

### Supplementary Note 4

The panel circuit according to Supplementary Note 2 or 3,
wherein the first groove portion has a portion extending in a first direction and a portion extending in a second direction intersecting the first direction.

### Supplementary Note 5

The panel circuit according to any one of Supplementary Notes 2 to 4,
wherein the second groove portion has a portion extending in a first direction and a portion extending in a second direction intersecting the first direction.

### Supplementary Note 6

The panel circuit according to any one of Supplementary Notes 2 to 5,
wherein the second groove portion has a larger width than the first groove portion.

### Supplementary Note 7

The panel circuit according to any one of Supplementary Notes 2 to 6,
wherein the second groove portion has a greater depth than the first groove portion.

### Supplementary Note 8

The panel circuit according to any one of Supplementary Notes 1 to 7,
wherein a thickness of the resin panel is 10 mm or less.

### Supplementary Note 9

The panel circuit according to any one of Supplementary Notes 1 to 8,
wherein the resin panel has light transmittance.

### Supplementary Note 10

The panel circuit according to any one of Supplementary Notes 1 to 9, further comprising:
at least one cell processing member connected to the tube.

### Supplementary Note 11

The panel circuit according to Supplementary Note 10,
wherein the cell processing member includes any one or more of a filter, a centrifuge tube, a bag, or a bottle.

### Supplementary Note 12

A cell processing device comprising:
the panel circuit according to any one of Supplementary Notes 1 to 11;
at least one pump that performs feeding of a liquid flowing inside the tube;
at least one pinch valve that switches between closing and opening of the tube; and
a liquid sensor that senses the liquid flowing inside the tube.

### Supplementary Note 13

The cell processing device according to Supplementary Note 12,
wherein the pump, the pinch valve, and the liquid sensor are provided on the same surface of a housing surface, and
the resin panel has a plurality of hole portions and is attached to the housing surface in a state in which any one of the pump, the pinch valve, or the liquid sensor is inserted into any of the plurality of hole portions.

### Explanation of References

- 10:: tube assembly
- 11:: tube
- 12:: filter
- 13:: centrifuge tube
- 14:: bag
- 15:: bottle
- 20, 20X:: resin panel
- 21:: first groove portion
- 22:: second groove portion
- 24, 25:: hole portion
- 30:: panel circuit
- 40:: cell processing device
- 41:: housing
- 42:: pump
- 43:: pinch valve
- 44:: liquid sensor
- 45:: pressure gauge
- 46:: positioning shaft

## Claims

1. A panel circuit comprising:
a resin panel having a first groove portion on a first surface;
a flexible tube that is fitted into the first groove portion to be guided by the first groove portion,
wherein a portion of a second surface of the resin panel opposite to the first surface, which corresponds to the first groove portion, protrudes from the other portion of the second surface, and
a most part of the tube is fitted into the first groove portion.

2. The panel circuit according to claim 1,
wherein the resin panel further has a second groove portion into which the tube is not fitted.

3. The panel circuit according to claim 2,
wherein the first groove portion and the second groove portion have a bent portion or a curved portion.

4. The panel circuit according to claim 2 or 3,
wherein the first groove portion has a portion extending in a first direction and a portion extending in a second direction intersecting the first direction.

5. The panel circuit according to any one of claims 2 to 4,
wherein the second groove portion has a portion extending in a first direction and a portion extending in a second direction intersecting the first direction.

6. The panel circuit according to any one of claims 2 to 5,
wherein the second groove portion has a larger width than the first groove portion.

7. The panel circuit according to any one of claims 2 to 6,
wherein the second groove portion has a greater depth than the first groove portion.

8. The panel circuit according to any one of claims 1 to 7,
wherein a thickness of the resin panel is 10 mm or less.

9. The panel circuit according to any one of claims 1 to 8,
wherein the resin panel has light transmittance.

10. The panel circuit according to any one of claims 1 to 9, further comprising:
at least one cell processing member connected to the tube.

11. The panel circuit according to claim 10,
wherein the cell processing member includes any one or more of a filter, a centrifuge tube, a bag, or a bottle.

12. A cell processing device comprising:
the panel circuit according to any one of claims 1 to 11;
at least one pump that performs feeding of a liquid flowing inside the tube;
at least one pinch valve that switches between closing and opening of the tube; and
a liquid sensor that senses the liquid flowing inside the tube.

13. The cell processing device according to claim 12,
wherein the pump, the pinch valve, and the liquid sensor are provided on the same surface of a housing surface, and
the resin panel has a plurality of hole portions and is attached to the housing surface in a state in which any one of the pump, the pinch valve, or the liquid sensor is inserted into any of the plurality of hole portions.
